(19) Europäisches Patentamt
European Patent Office
Office européen des brevets

(11) Veröffentlichungsnummer: **0 582 916 A1**

# (12) EUROPÄISCHE PATENTANMELDUNG

(21) Anmeldenummer: **93112258.4**

(51) Int. Cl.[5]: **C07D 215/14**, A61K 31/47

(22) Anmeldetag: **30.07.93**

(30) Priorität: **12.08.92 DE 4226649**

(43) Veröffentlichungstag der Anmeldung:
**16.02.94 Patentblatt 94/07**

(84) Benannte Vertragsstaaten:
**AT BE CH DE DK ES FR GB GR IE IT LI LU MC NL PT SE**

(71) Anmelder: **BAYER AG**

**D-51368 Leverkusen(DE)**

(72) Erfinder: **Raddatz, Siegfried**
**Jakob-Böhme-Strasse 21**
**D-51065 Köln(DE)**
Erfinder: **Mohrs, Klaus-Helmut**
**Wildsteig 24**
**D-42113 Wuppertal(DE)**
Erfinder: **Matzke, Michael**
**Am Ringelbusch 15**
**D-42113 Wuppertal(DE)**
Erfinder: **Fruchtmann, Romanis**
**Weidenpescher Strasse 14**
**D-50735 Köln(DE)**
Erfinder: **Müller-Peddinghaus, Reiner, Prof. Dr.**
**Klutstein 22a**
**D-51467 Bergisch Gladbach(DE)**
Erfinder: **Hatzelmann, Armin**
**Alter Wall 3**
**D-78467 Konstanz(DE)**

## (54) N-Methansulfonyl-2- 3-isobutyl-3-(chinolin-2-yl-methoxy)phenyl -2-cycloalkylessigsäureamide, Verfahren zu ihrer Herstellung und ihre Verwendung in Arzneimitteln.

(57) Isobutylsubstituierte Methansulfonyl-chinolylmethoxyphenyl-cycloalkylessigsäureamide der Formel

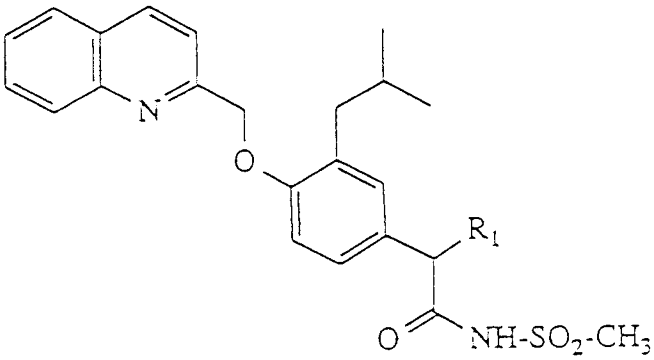

in welcher
$R^1$ für Cyclohexyl steht
in Form des Racemats und der Enantiomeren
sowie die Enantiomeren
wenn
$R^1$ für Cyclopentyl oder Cycloheptyl steht
und deren Salze.

EP 0 582 916 A1

Die isobutylsubstituierten Methansulfonylchinolylmethoxyphenyl-cycloalkylessigsäureamide können hergestellt werden durch Umsetzung der entsprechenden racemischen oder enantiomeren Säuren mit Methansulfonamid, wobei die racemischen Endprodukte nach üblichen Methoden in die Enantiomeren getrennt werden können. Die N-Methansulfonyl-2-[3-isobutyl-3-(chinolin-2-yl-methoxy)phenyl]-2-cycloalkylessigsäureamide können als Wirkstoffe in Arzneimitteln eingesetzt werden. Die Stoffe können als Hemmer von enzymatischen Reaktionen im Rahmen des Arachidonsäurestoffwechsels, insbesondere der Lipoxygenase, wirken.

Die Erfindung betrifft neue isobutylsubstituierte Methansulfonyl-chinolylmethoxyphenyl-cycloalkylessigsäureamide, ein Verfahren zu ihrer Herstellung und ihre Verwendung in Arzneimitteln.

Substituierte 4-(Chinolin-2-yl-methoxy)phenylessigsäurederivate und α-substituierte 4-(Chinolin-2-yl-methoxy)phenylessigsäurederivate sind aus EP 344 519 (US 4 970 215) und EP 339 416 bekannt.

Die vorliegende Erfindung betrifft neue isobutylsubstituierte Methansulfonyl-chinolylmethoxyphenyl-cycloalkylessigsäureamide der allgemeinen Formel (I)

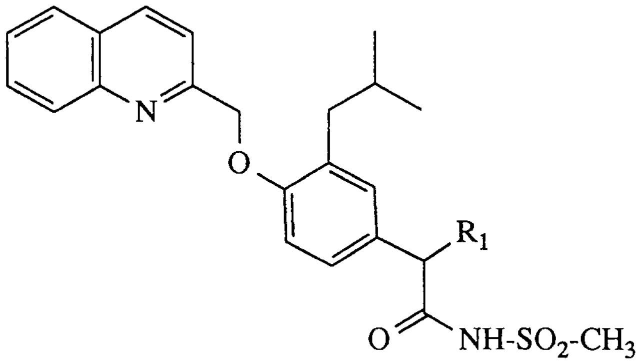

(I)

in welcher

$R^1$ für Cyclohexyl steht,

in Form des Racemats und der Enantiomeren

sowie die beiden Enantiomeren der Verbindungen der Formel (I) in denen $R^1$ für Cyclopentyl oder Cycloheptyl steht

und deren Salze.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Metallsalze, bevorzugt der einwertigen Metalle, und die Ammoniumsalze. Bevorzugt werden Alkalisalze wie beispielsweise Natrium-, Kalium- und Ammoniumsalze.

Die erfindungsgemäßen Verbindungen der allgemeinen Formel (I) werden hergestellt, indem man die Carbonsäuren der allgemeinen Formel (II)

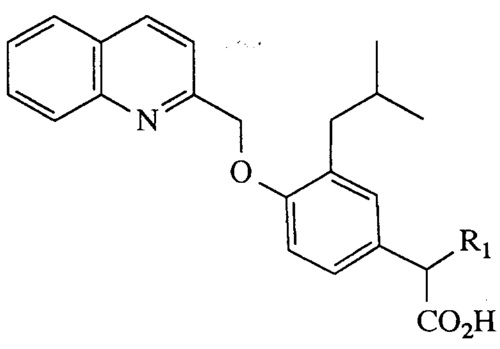

(II)

in welcher

$R^1$ die oben angegebene Bedeutung hat,

gegebenenfalls unter vorgeschalteter Aktivierung, mit Methansulfonamid der Formel (III)

$H_2N\text{-}SO_2\text{-}CH_3$ (III)

in inerten Lösemitteln, in Anwesenheit einer Base und/oder Katalysator umsetzt, und im Fall der Enantiomeren, entweder direkt die enantiomerenreinen Säuren (II) einsetzt oder die Racemate (I) nach üblichen Methoden trennt.

Die Trennung der Enantiomeren erfolgt bevorzugt säulenchromatographisch.

Das erfindungsgemäße Verfahren kann durch folgendes Formelschema beispielhaft erläutert werden:

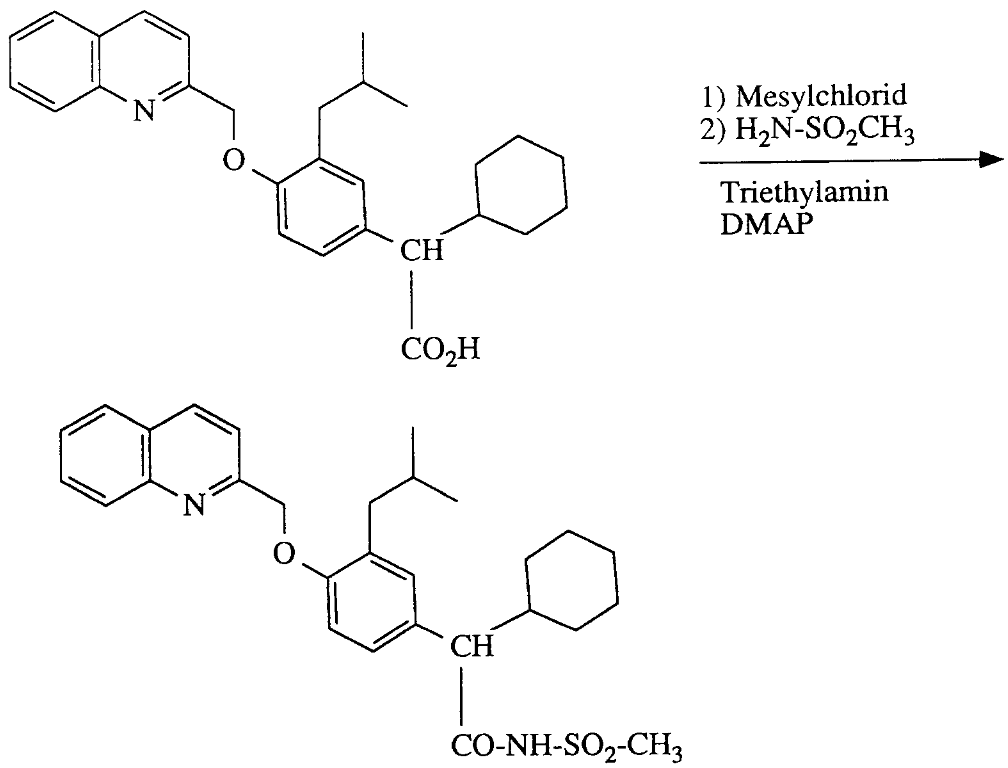


Die Sulfoamidierung erfolgt im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwassserstoffe wie Benzol, Xylol, Toluol, Hexan, Cyclohexan, oder Erdölfraktionen, Nitromethan, Dimethylformamid, Acetonitril oder Hexamethylphosphorsäuretriamid. Ebenso ist es möglich, Gemische der Lösemittel einzusetzen. Besonders bevorzugt ist Dichlormethan.

Als Basen für die Sulfoamidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.buylat, oder organische Amine wie Benzyltrimethylammoniumhydroxid, Tetrabutylammoniumhydroxid, Dimethylaminopyridin, Pyridin, Triethylamin oder N-Methylpiperidin.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphonsäurediphenylesteramid oder Methansulfonsäurechlorid, gegebenenfalls in Anweserheit von Basen wie Triethylamin oder N-Ethylmorpholin oder N-Methylpiperidin oder Dicyclohexylcarbodiimid und N-Hydroxysuccinimid.

Bei der Durchführung der Sulfoamidierung wird die Base im allgemeinen in einer Menge von 1 bis 3 Mol, bevorzugt von 1 bis 1,5 Mol, bezogen auf 1 Mol der Carbonsäure (II), eingesetzt.

Im Fall der Umsetzung über das gemischte Anhydrid wird als Katalysator bevorzugt Dimethylaminopyridin eingesetzt.

Der Katalysator wird im allgemeinen in katalytischen bis äquimolaren, bevorzugt äquimolaren Mengen, eingesetzt.

Die Sulfoamidierung wird im allgemeinen in einem Temperaturbereich von 0 °C bis 150 °C, bevorzugt bei 25 °C bis 40 °C, durchgeführt.

Die Sulfoamidierung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Die Carbonsäuren der allgemeinen Formel (II) sind mit Ausnahme des Falls $R^1$ = Cycloheptyl, als konkrete Stoffvertreter, insbesondere in enantiomerenreiner Form, neu und können beispielsweise hergestellt werden, indem man
entweder Verbindungen der allgemeinen Formel (IV) oder (IVa)

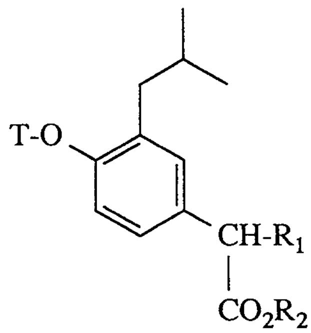

(IV) oder

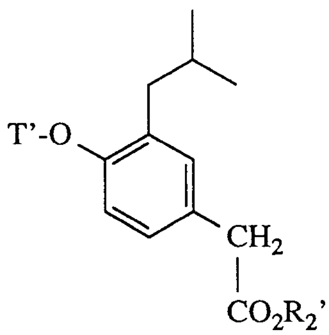

(IVa)

in welchen

$R^1$ die oben angegebene Bedeutung hat,

T und T' gleich oder verschieden sind und für eine Hydroxyschutzgruppe wie Benzyl oder tert.Butyl stehen

und

$R^2$ und $R^{2'}$ gleich oder verschieden sind und $C_1$-$C_4$-Alkyl bedeuten,

nach Abspaltung der Schutzgruppe mit 2-Halogenmethylchinolin der Formel (V)

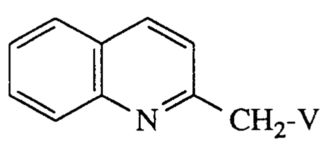

(V)

in welcher

V für Halogen, bevorzugt für Chlor oder Brom steht,

in inerten Lösemitteln durch Veretherung in die Verbindungen der allgemeinen Formel (VI) oder (VIa)

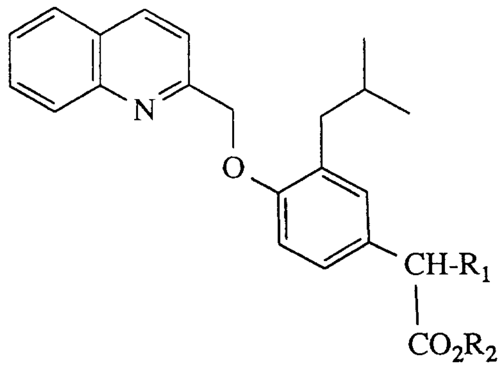

(VI) oder

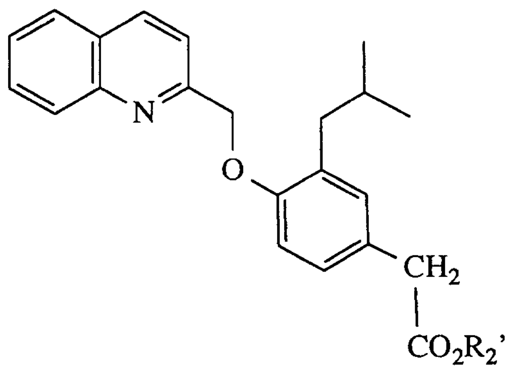

(VIa)

in welchen

$R^1$, $R^2$ und $R^{2'}$ die oben angegebene Bedeutung haben,

überführt

und im Fall der Verbindungen der allgemeinen Formel (VIa) diese in einem 2. Schritt einer Alkylierung mit Verbindungen der allgemeinen Formel (VII)

$R^1$-W (VII),

in welcher

$R^1$ die oben angegebene Bedeutung hat

und

W für Chlor, Brom oder Jod steht,

in inerten Lösemitteln umsetzt,

und anschließend die Ester nach üblichen Methoden verseift,

und im Fall der Enantiomere, die racemischen Säuren nach üblicher Methode an chiralen Säuren säulenchromatographisch trennt.

Die Abspaltung der Schutzgruppen aus den entsprechenden Ethern (IV) und (IVa) erfolgt nach üblicher Methode, beispielsweise durch hydrogenolytische Spaltung der Benzylether in den oben aufgeführten inerten Lösemitteln in Anwesenheit eines Katalysators mit Wasserstoff-Gas.

Die Veretherung kann in inerten organischen Lösemitteln gegebenenfalls in Anwesenheit einer Base durchgeführt werden. Lösemittel für die Veretherung können inerte organische Lösemittel sein, die sich unter den Reaktionsbedingungen nicht verändern. Hierzu gehören vorzugsweise Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische der Lösemittel einzusetzen.

Als Basen für die Veretherung können anorganische oder organische Basen eingesetzt werden. Hierzu gehören vorzugsweise Alkalihydroxide wie z.B. Natriumhydroxid oder Kaliumhydroxid, Erdalkalihydroxide wie z.B. Bariumhydroxid, Alkalicarbonate wie Natriumcarbonat oder Kaliumcarbonat, Erdalkalicarbonate wie Calciumcarbonat, oder organische Amine (Trialkyl($C_1$-$C_6$)amine) wie Triethylamin, oder Heterocyclen wie

Pyridin, Methylpiperidin, Piperidin oder Morpholin.

Es ist auch möglich, als Basen Alkalimetalle wie Natrium und deren Hydride, wie Natriumhydrid, einzusetzen.

Die Veretherung erfolgt im allgemeinen in einem Temperaturbereich von 0 °C bis +150 °C, bevorzugt von +10 °C bis +100 °C.

Die Veretherung wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5 Mol, bevorzugt 1 bis 2 Mol Halogenid (V, bezogen auf 1 Mol des Reaktionspartners, ein. Die Base wird im allgemeinen in einer Menge von 0,5 bis 5 Mol, bevorzugt von 1 bis 3 Mol, bezogen auf das Halogenid, eingesetzt.

Die Verbindungen der allgemeinen Formel (IV) und (IVa) sind an sich bekannt oder können nach üblicher Methode hergestellt werden.

Ebenfalls sind die Verbindungen der allgemeinen Formel (V) und ihre Darstellung bekannt.

Als Lösemittel für die erfindungsgemäßen Verfahren und für die Alkylierung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Die Alkylierung wird in den oben aufgeführten Lösemitteln bei Temperaturen von 0 °C bis +150 °C, vorzugsweise bei Raumtemperaturen bis +100 °C, bei Normaldruck durchgeführt.

Die Verbindungen der allgemeinen Formel (III) sind an sich bekannt.

Die Verbindungen der allgemeinen Formel (VI) sind als konkrete Stoffvertreter teilweise neu und können wie oben beschrieben hergestellt werden.

Die Trennung der Racemate erfolgt säulenchromatographisch im allgemeinen an chiralen HPLC-Säulen mit Lösemittelgemischen wie beispielsweise n-Heptan / 2-Propanol oder über diastereomere Ester.

Die erfindungsgemäßen neuen N-Methansulfonyl-2-[3-isobutyl-3-(chinolin-2-yl-methoxy)phenyl]-2-cycloalkylessigsäureamide können als Wirkstoffe in Arzneimitteln eingesetzt werden. Die Stoffe können als Hemmer von enzymatischen Reaktionen im Rahmen des Arachidonsäurestoffwechsels, insbesondere der Lipoxygenase, wirken.

Sie sind somit bevorzugt zur Behandlung und Verhütung von Erkrankungen der Atemwege wie Allergien/Asthma, Bronchitis, Emphysem, Schocklunge, pulmonaler Hypertonie, Entzündungen/Rheuma und Ödemen, Thrombosen und Thromboembolien, Ischämien (periphere, cardiale, cerebrale Durchblutungsstörungen), Herz-und Hirninfarkten, Angina pectoris, Arteriosklerose, bei Gewebstransplatationen, Dermatosen wie Psoriasis, entzündliche Dermatosen und zur Cytoprotection im Gastrointestinaltrakt geeignet.

Die erfindungsgemäßen phenylsubstituierten Chinoline können sowohl in der Humanmedizin als auch in der Veterinärmedizin angewendet werden.

Die pharmakologischen Wirkungen der erfindungsgemäßen Substanzen werden durch folgende Methode bestimmt:

Als Maß für die Lipoxyenase-Hemmung wurde die Freisetzung von Leukotrien $B_4$ ($LTB_4$) an polymorphkernigen Humanleukozyten (PMN) nach Zugabe von Substanzen und Ca-Ionophor mittels reverse phase HPLC nach Borgeat, P. et al., Proc. Nat. Acad. Sci, 76, 2148 - 2152 (1979), bestimmt.

Lipoxygenasehemmung (Human PMNL)

| Bsp.-Nr. | $IC_{50}$[mol/l] |
|---|---|
| 1 | $5,2 \times 10^{-8}$ |
| 6 | $5,8 \times 10^{-8}$ |
| 7 | $7,0 \times 10^{-8}$ |

Zur vorliegenden Erfindung gehören auch pharmazeutische Zubereitungen, die neben inerten, nichttoxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Ausgangsverbindungen

Beispiel I

2-[3-Isobutyl-4-(chinolin-2-yl-methoxy)phenyl]-2-cyclohexyl-essigsäuremethylester

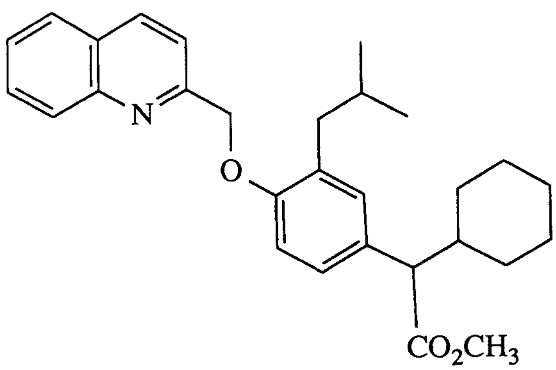

Unter Argonatmosphäre werden 12 g (0,033 mol) 2-[3-Isobutyl-4-(chinolin-2-yl-methoxy)-phenylessigsäuremethylester und 6,52 g (0,04 mol) = 4,9 ml Cyclohexylbromid in 36 ml DMF gelöst und auf 0 °C abgekühlt. Unter Rühren tropft man dazu 4,88 g (0,04 mol) Kaliumtertiärbutylat, gelöst in 80 ml DMF. Nach ca. 2 h Reaktionszeit läßt man die Temperatur auf RT ansteigen, säuert mit 2 n Salzsäure an und engt i.V. zur Trockne ein. Man rührt den verbleibenden Rest mit 100 ml Dichlormethan und 50 ml Wasser aus, trennt die organische Phase ab, trocknet sie mit $Na_2SO_4$, engt i.V. auf ein kleines Volumen ein und trennt den verbleibenden Rest säulenchromatographisch (Kieselgel 60, Laufmittel: Dichlormethan / Essigester = 100/2).

Ausbeute: 13 g (88,4% d.Th.), leicht gelbliches Öl

Beispiel II

2-[3-Isobutyl-4-(chinolin-2-yl-methoxy)phenyl]-2-cyclohexyl-essigsäure

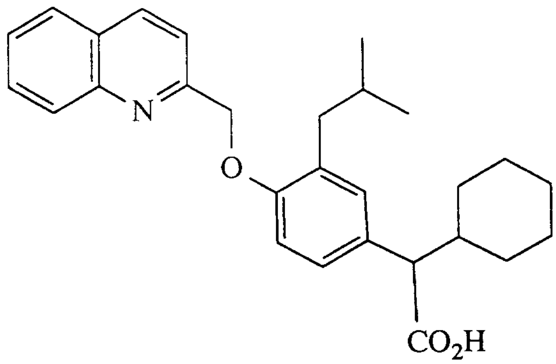

10,2 g (0,0236 mol) der Verbindung aus Beispiel I werden in 70 ml 2-Propanol aufgenommen und mit 50 ml 1 n Natronlauge über Nacht zum Sieden erhitzt. Nach dem Erkalten neutralisiert man mit 50 ml 1 n Salzsäure. Den angefallenen Niederschlag saugt man ab, wäscht und trocknet ihn und rekristallisiert anschließend aus Diisopropylether.
Ausbeute: 9,5 g (96,3% d.Th.), farblose Kristalle
Fp.: 130 °C

Beispiel III und Beispiel IV

(+) 2-[3-Isobutyl-4-(chinolin-2-yl-methoxy)phenyl]-2-cyclohexyl-essigsäure (Beispiel III)
(-) 2-[3-Isobutyl-4-(chinolin-2-yl-methoxy)phenyl]-2-cyclohexyl-essigsäure (Beispiel IV)

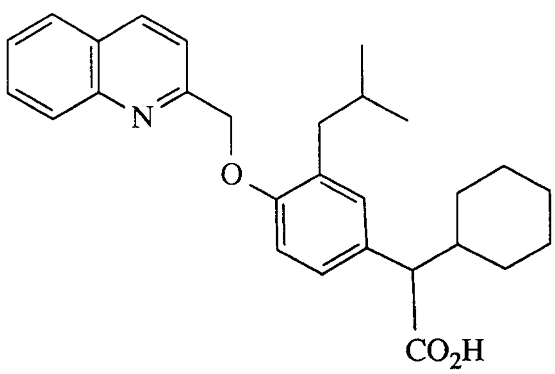

Die Titelverbindungen werden durch Trennung des Racemates (Beispiel II) durch chromatographische Trennung an chiralen Säulen erhalten.

| | |
|---|---|
| (+)-Enantiomer: | spez. Dreh.: 17,96 ($CHCl_3$)(Beispiel III) |
| | mol. Dreh.: 77,41 |
| (-)-Enantiomer: | spez. Dreh.: - 18,86 ($CHCl_3$)(Beispiel IV) |
| | mol. Dreh.: - 81,28 |

Beispiel V

2-[3-Isobutyl-4-(chinolin-2-yl-methoxy)phenyl]-2-cycloheptylessigsäuremethylester

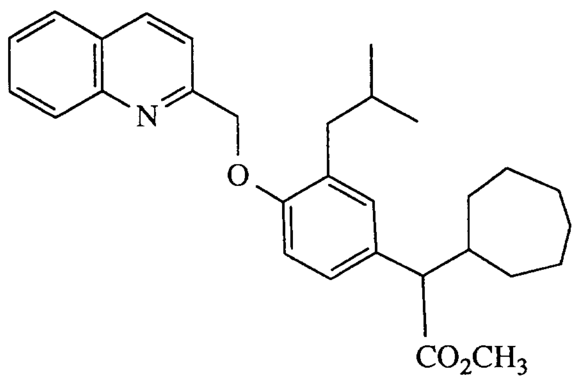

10 g (0,0275 mol) 2-[3-Isobutyl-4-(chinolin-2-yl-methoxy)phenylessigsäuremethylester werden in Analogie zur Vorschrift des Beispiels I mit 10,04 g (0,055 mol) Cycloheptylbromid und 6,17 g (0,055 mol) Kaliumtertiärbutylat zur Titelverbindung umgesetzt.
Ausbeute: quantitativ, gelbbraunes Öl

Beispiel VI

2-[3-Isobutyl-4-(chinolin-2-yl-methoxy)phenyl]-2-cycloheptylessigsäure

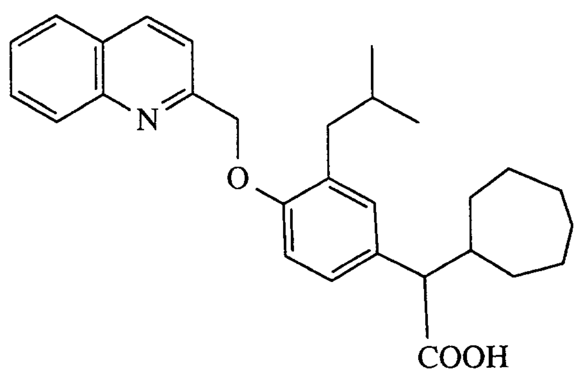

In Analogie zur Vorschrift des Beispiels II wird die Titelverbindung aus der Verbindung des Beispiels V und 30 ml 1n Natronlauge mit anschließendem Ansäuern hergestellt.
Ausbeute: 11,3 g (92,3% d.Th.), farblose Kristalle
Fp.: 112°C

Beispiel VII und Beispiel VIII

(+) 2-[3-Isobutyl-4-(chinolin-2-yl-methoxy)phenyl]-2-cycloheptylessigsäure (Beispiel VII)
(-) -2-[3-Isobutyl-4-(chinolin-2-yl-methoxy)phenyl]-2-cycloheptylessigsäure (Beispiel VIII)

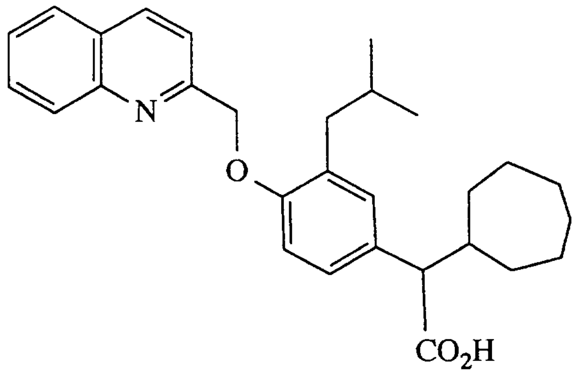

Die Titelverbindungen werden aus dem Racemat (Beispiel VI) durch chromatographische Trennung an HPLC-H 1050-Chiralpak AS in einem Lösemittelgemisch 96% n-Heptan und 4% eines Gemisches von 2-Propanol, das 1% Wasser und 0,2% Trifluoressigsäure enthält, hergestellt.

| | |
|---|---|
| (+)-Enantiomer: | spez. Dreh.: 15,72, Lösemittel $CHCl_3$, Beispiel VII |
| | mol. Dreh.: 69,96 |
| (-)-Enantiomer: | spez. Dreh.: -18,7, Lösemittel $CHCl_3$, Beispiel VIII |
| | mol. Dreh.: -86,19 |

Beispiel IX

2-[3-Isobutyl-4-(chinolin-2-yl-methoxy)phenyl]-2-cycloheptylessigsäuremethylester

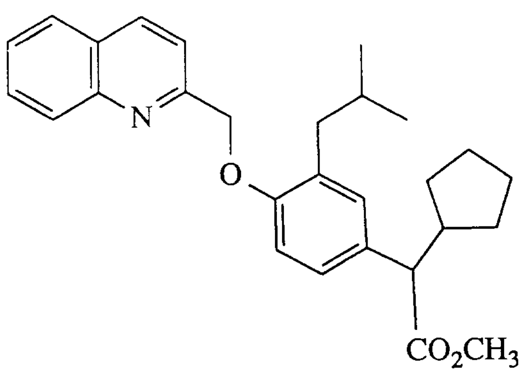

In Analogie zu den Vorschriften der Beispiele I und V wird die Titelverbindung aus 10 g (0,0275 mol) 2-[3-Isobutyl-4-(chinolin-2-yl-methoxy)phenyl]-essigsäuremethylester, 8,2 g (0,055 mol) Cyclopentylbromid und 6,17 g (0,055 mol) Kaliumtertiärbutylat hergestellt.
Ausbeute: quantitativ, gelbbraunes Öl

Beispiel X

2-[3-Isobutyl-4-(chinolin-2-yl-methoxy)phenyl]-2-cyclopentylessigsäure

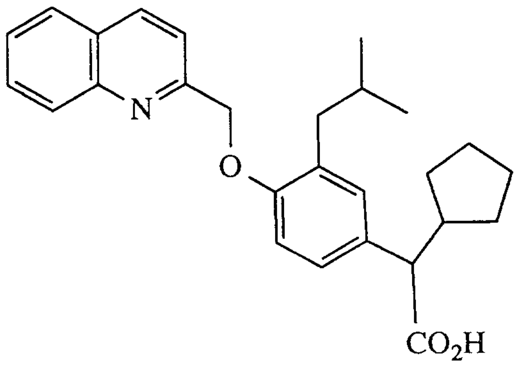

In Analogie zu den Vorschriften der Beispiele II und VI wird die Titelverbindung aus der Verbindung des Beispiels IX durch Verseifung mit 30 ml Natronlauge und anschließendem Ansäueren hergestellt.
Ausbeute: 10,5 g (91,5% d.Th.), leicht gelbliche Kristalle
F: 120 °C

Beispiel XI und XII

(+)-2-[3-Isobutyl-4-(chinolin-2-yl-methoxy)phenyl]-2-cyclopentylessigsäure (Beispiel XI)
(-) -2-[3-Isobutyl-4-(chinolin-2-yl-methoxy)phenyl]-2-cyclopentylessigsäure (Beispiel XII)

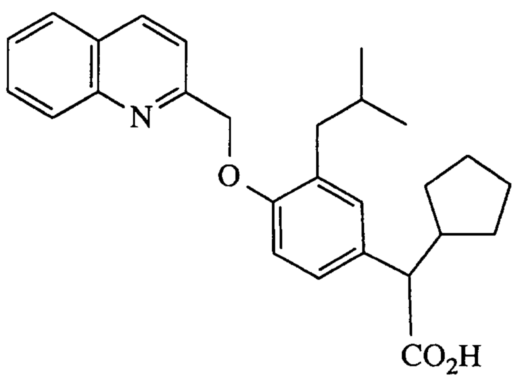

Die Titelverbindungen werden in Analogie zu der Vorschrift der Beispiele VII und VIII durch chromatographische Trennung der Verbindung des Beispiels X erhalten.

| | |
|---|---|
| (+)-Enantiomer: | spez. Dreh.: 44,56 (THF), Beispiel XI |
| | mol. Dreh.: 185,84 |
| (-)-Enantiomer: | spez. Dreh.: -41,07 (THF), Beispiel XII |
| | mol. Dreh.: -171,28 |

Beispiel XIII

N-Methansulfonyl-2-[3-isobutyl-4-(chinolin-2-yl-methoxy)phenyl]-2-cycloheptylessigsäureamid

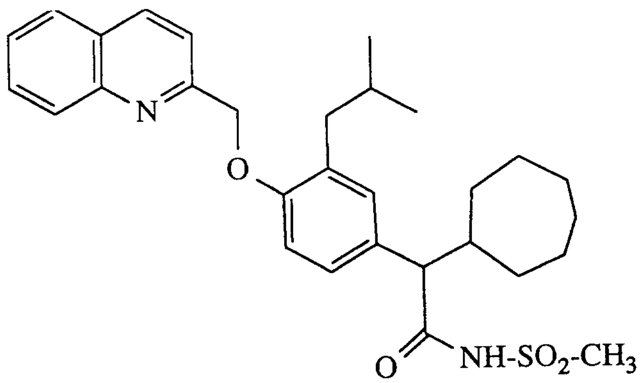

Unter Argon werden 2 g (0,0045 mol) der Verbindung aus Beispiel VI in 20 ml getrocknetem THF aufgeschlämmt und mit 1,82 g (0,018 mol) Triethylamin versetzt (d = 0,73). Dabei entsteht eine klare Lösung. Bei Eisbadkühlung tropft man 1,14 g (0,01 mol) Mesylchlorid (d = 1,48) zu, rührt bei dieser Temperatur noch 15 min nach und läßt dann unter Rühren 1,52 g (0,016 mol, Methansulfonamid und 1,1 g (0,009 mol) Dimethylaminopyridin, gelöst in 10 ml trockenem THF, zutropfen. Man läßt über Nacht nachreagieren, wobei die Temperatur auf RT ansteigt. Der Ansatz wird auf Toluol gegossen und mit Wasser und verdünnter Essigsäure extrahiert. Die organische Phase trennt man ab, trocknet mit $Na_2SO_4$, engt im Vakuum auf ein kleines Volumen ein und säult das verbleibende hellbraune Öl (2,42 g) säulenchromatographisch (Kieselgel 60, Laufmittel: Toluol / Essigester / Eisessig = 8/2/1).
Man erhält 1,75 g (74,6% d.Th,) eines farbloses Produkts (amorph.)

Herstellungsbeispiele

Beispiel 1

N-Methansulfonyl-2-[3-isobutyl-4-(chinolin-2-yl-methoxy)phenyl]-2-cyclohexylessigsäureamid

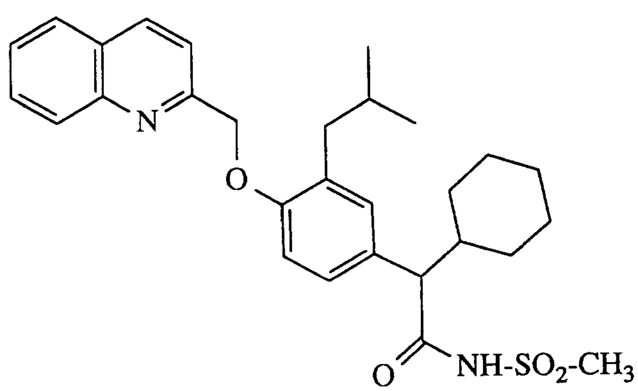

Die Titelverbindung wird in Analogie zur Vorschrift des Beipiels XIII aus 3,5 g (0,008 mol) der Verbindung aus Beispiel II, 1 g (0,008 mol) Mesylchlorid, 0,912 g Methansulfonamid, 1,62 g (0,016 mol) Triehylamin und 0,98 g (0,008 mol) Dimethylaminopyridin hergestellt.
Ausbeute: 3,48 g (84,9% d.Th.), farbloses amorphes Pulver
F: 163-170 °C

Beispiel 2 und Beispiel 3

(+) -N-Methansulfonyl-2-[3-isobutyl-4-(chinolin-2-yl-methoxy)phenyl]-2-cyclohexylessigsäureamid (Beispiel 2)
(-) -N-Methansulfonyl-2-[3-isobutyl-4-(chinolin-2-yl-methoxy)phenyl]-2-cyclohexylessigsäureamid (Beispiel 3)

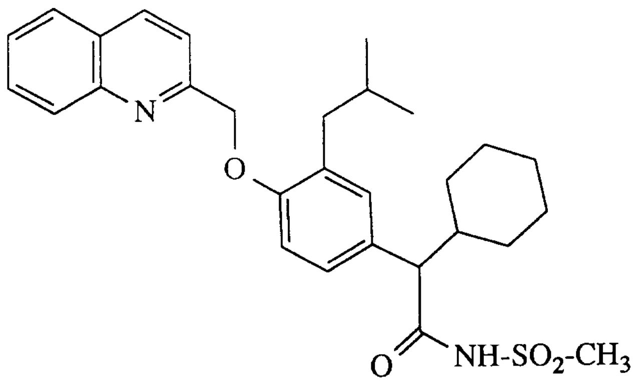

Die beiden Enantiomere werden durch säulenchromatographische Trennung der Verbindung aus Beispiel 1 an HPLC-HP 1050 Chiralcel OD in einem Laufmittelgemisch von 86% n-Heptan und 14% eines 2-Propanolgemisches, das 1% Wasser + 0,2% Trifluoressigsäure enthält, erhalten.

| | |
|---|---|
| (+)-Enantiomer: | spez. Dreh.: +32,15° ($CHCl_3$) Beispiel 2 |
| | mol. Dreh.: +163,32° |
| (-)-Enantiomer: | spez. Dreh.: -28,96° ($CHCl_3$) Beispiel 3 |
| | mol. Dreh.: -147,12° |

Die in Tabelle 1 aufgeführten reinen Enantiomere können entweder in Analogie zu den Vorschriften der Beispiele 2 und 3 über Trennung des Racemats oder durch Einsatz der entsprechenden enantiomerenreinen Carbonsäuren hergestellt werden.

Tabelle 1:

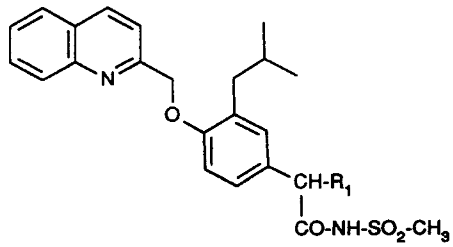

| Bsp.-Nr. | $R^1$ | Enantiomer | mol. Dreh. | spez. Dreh. |
|---|---|---|---|---|
| 4 | 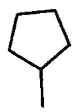 | (+) | | |
| 5 | 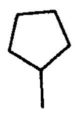 | (-) | | |
| 6 | 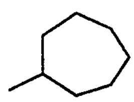 | (+) | +147,99° ($CHCl_3$) | + 28,35° ($CHCl_3$) |
| 7 | 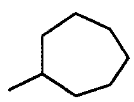 | (-) | -169,91° ($CHCl_3$) | -32,55° ($CHCl_3$) |

## Patentansprüche

**1.** Neue isobutylsubstituierte Methansulfonyl-chinolylmethoxyphenyl-cycloalkylessigsäureamide der Formel

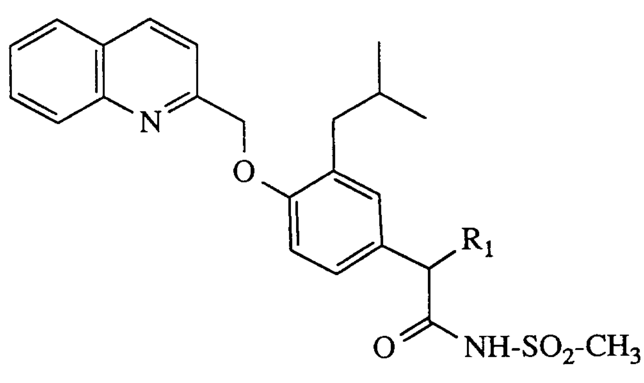

in welcher

$R^1$ für Cyclohexyl steht

in Form des Racemats und der Enantiomeren

sowie die Enantiomeren

wenn
R[1] für Cyclopentyl oder Cycloheptyl steht
und deren Salze.

**2.** Racemat, (+)-Enantiomer und (-)-Enantiomer der Verbindung der Formel

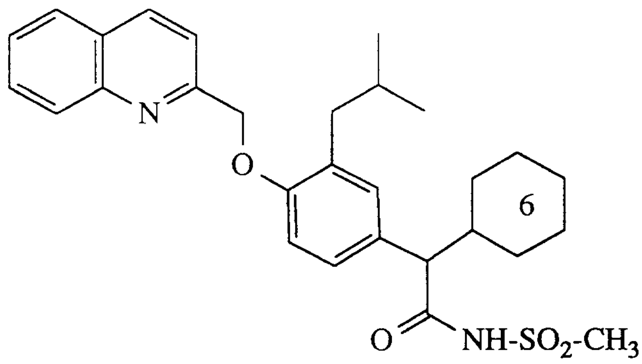

und deren Salze.

**3.** (+)-Enantiomer und (-)-Enantiomer der Verbindung der Formel

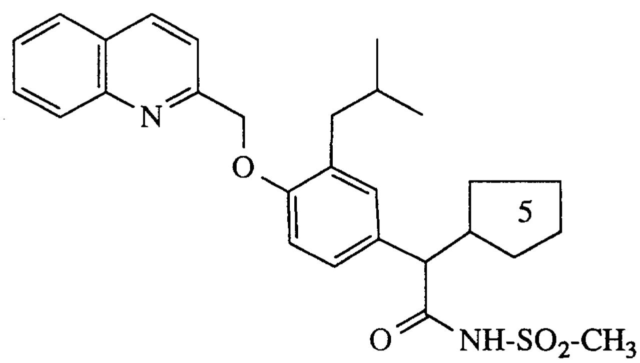

und deren Salze.

**4.** (+)-Enantiomer und (-)-Enantiomer der Verbindung der Formel

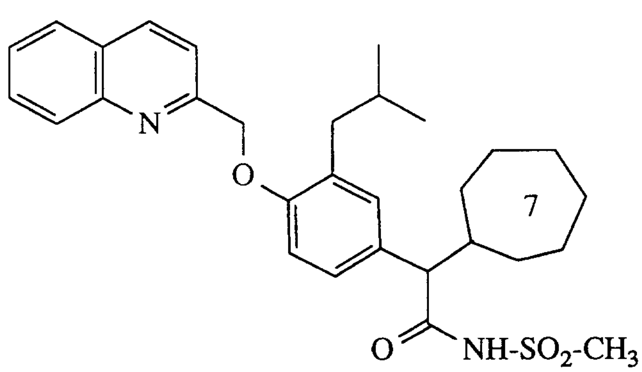

und deren Salze.

5. Neue isobutylsubstituierte Methansulfonyl-chinolylmethoxyphenyl-cycloalkylessigsäureamide nach Anspruch 1 zur therapeutsichen Anwendung.

6. Verfahren zur Herstellung von neuen isobutylsubstituierten Methansulfonylchinolylmethoxy-cycloalkylphenylessigsäureamiden nach Anspruch 1, dadurch gekennzeichnet, daß man Carbonsäuren der allgemeinen Formel (II)

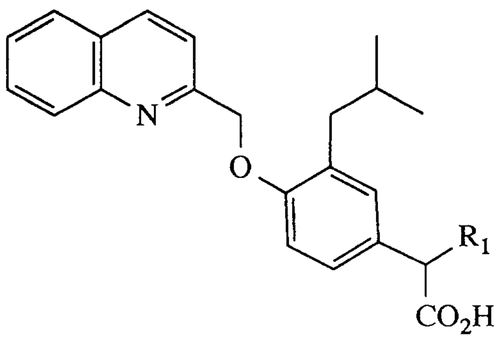

(II)

in welcher

$R^1$ die oben angegebene Bedeutung hat,

gegebenenfalls unter vorgeschalteter Aktivierung, mit Methansulfonamid der Formel (III)

$H_2N\text{-}SO_2\text{-}CH_3$ (III)

in inerten Lösemitteln, in Anwesenheit einer Base und/oder Katalysator umsetzt,
und im Fall der Enantiomeren, entweder direkt die enantiomerenreinen Säuren (II) einsetzt oder die Racemate (I) nach üblichen Methoden trennt.

7. Verfahren nach Anspruch 6, dadurch gekennzeichnet, daß man die Enantiomeren säulenchromatographisch trennt.

8. Arzneimittel enthalten mindestens ein isobutylsubstituiertes Methansulfonylchinolylmethoxyphenyl-cycloalkylessigsäureamid nach Anspruch 1.

9. Verwendung von isobutylsubstitutierten Methansulfonyl-chinolylmethoxyphenyl-cycloalkylessigsäureamiden nach Anspruch 1 zur Herstellung von Arzneimitteln.

10. Verwendung nach Anspruch 9 zur Herstellung von lipoxygenasehemmenden Arzneimitteln

Europäisches Patentamt

# EUROPÄISCHER RECHERCHENBERICHT

Nummer der Anmeldung

EP 93 11 2258

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| P,X | EP-A-0 499 926 (BAYER AG)<br>26. August 1992<br>* Beispiele 66,75; Seite 3, Zeilen 12-17 *<br>* Beispiele 68,69,70; Verfahren [B], Seite 6 und 8 *<br>* Ansprüche 1-10 *<br>--- | 1,3,4,5, 7-10 | C07D215/14<br>A61K31/47 |
| P,A | EP-A-0 509 359 (BAYER AG)<br>21. Oktober 1992<br>* Ansprüche 1-7; Beispiele 3,4,7,8 *<br>--- | 1-10 | |
| A | EP-A-0 399 291 (BAYER AG)<br>28. November 1990<br>* Ansprüche 11,12,13; Beispiele 1,9,13 *<br>--- | 1-10 | |
| D,A | EP-A-0 344 519 (BAYER AG)<br>6. Dezember 1989<br>* Beispiele 34A,34B,35,36 *<br>----- | 1-10 | |
| | | | RECHERCHIERTE SACHGEBIETE (Int. Cl.5 ) |
| | | | C07D<br>A61K |

Der vorliegende Recherchenbericht wurde für alle Patentansprüche erstellt

| Recherchenort | Abschlußdatum der Recherche | Prüfer |
|---|---|---|
| MUENCHEN | 08 OKTOBER 1993 | HARTRAMPF G.W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)